# EUROPEAN PATENT SPECIFICATION

(11) **EP 4 327 784 B1**
(45) Date of publication and mention of the grant of the patent: **31.12.2025**
(21) Application number: 23192491.1
(22) Date of filing: 21.08.2023
(51) Int. Cl.: A61F 2/38, A61F 2/30

(54) **TIBIAL PROSTHESIS WITH DISTAL FEATURES FOR CEMENTED FIXATION**
TIBIAPROTHESE MIT DISTALEN MERKMALEN ZUR ZEMENTIERTEN FIXIERUNG
PROTHÈSE TIBIALE AVEC ÉLÉMENTS DISTAUX POUR LA FIXATION CIMENTÉE

(30) Priority: 22.08.2022 US 202263399902 P
(43) Date of publication of application: 28.02.2024
(60) Divisional application: 24168856.3
(73) Proprietor: Zimmer, Inc., Warsaw, IN 46580 (US)
(72) Inventor: BISEK, Aaron J., Elk River, 55330 (US); TRISCHLER, Cory, Warsaw, 46580 (US); BRANSCOME, Douglas G., Fort Wayne, 46804 (US); WILSON, Vanessa Marie, Warsaw, 46580 (US)
(74) Representative: Taor, Simon Edward William

(56) References cited:
- DE-A1- 102015 109 353
- US-A- 4 298 992
- US-A- 4 938 769
- US-A1- 2016 278 929
- US-A1- 2019 282 368

## Description

### FIELD

The present subject matter relates to orthopedic prostheses and, more particularly, to tibial prostheses such as baseplates used in knee arthroplasties.

### BACKGROUND

Orthopedic procedures and prostheses are commonly utilized to repair and/or replace damaged bone and tissue in the human body. For example, a knee arthroplasty can be used to restore natural knee function by repairing damaged or diseased articular surfaces of the femur and/or tibia. An incision is made into the knee joint to expose the bones comprising the joint. Cut guides are used to guide the removal of the articular surfaces that are to be replaced. Prostheses are used to replicate the articular surfaces. Knee prostheses can include a femoral component implanted on the distal end of the femur, which articulates with a tibial bearing component and a tibial component (sometimes called a tibial tray or tibial baseplate) implanted on the proximal end of a tibia. Together these components replicate the function of a healthy natural knee. Various types of arthroplasties are known including a total knee arthroplasty, where all of the articulating compartments of the joint are repaired with prosthetic components.

US2019/282368A1 discloses an orthopaedic prosthesis including a tibial tray. The tibial tray includes a distal pocket and a plurality of inner pockets. Each inner pocket includes a channel sized to receive bone cement. The tibial tray includes distal-facing surfaces that have a surface roughness (Ra) equal to about 5.0 microns.

US4938769A discloses a tibial prosthesis for use during a total knee arthroplasty procedure which includes a modular tibial component comprising an inbone anchorage assembly to which is removably attached a tibial tray adapted to receive and retain a variety of femoral components or bearing inserts. Removal of the tray permits access to the interface between the bone and anchorage assembly in the event removal or revision are necessary.

### OVERVIEW

This disclosure pertains generally to tibial prostheses used in a knee arthroplasty including a total knee arthroplasty. The present inventors have recognized, among other things, a tibial baseplate including distal features that can facilitate better bonding with bone cement such that the tibial baseplate can be fixedly retained on the proximal tibia. Better bonding can reduce micro-motion of the tibial baseplate and can provide better overall durability for the tibial baseplate. Furthermore, present inventors have recognized the distal features can provide for a greater rigidity and torsional strength for the tibial baseplate. Additionally, the present inventors contemplate apparatuses and systems that allow a surgeon to prepare the tibia during surgery but allow the surgeon to choose intraoperatively between a cemented apparatus or a non-cemented apparatus. In particular, the cemented apparatus and the non-cemented apparatus can share a same or similar stock size and can share a same or similar tibial keel and/or fin geometry (e.g., a substantially same distal profile, shape, size, etc. in two or more dimensions). Systems of different stock sizes of the cemented apparatuses and different stock sizes of the non-cemented apparatuses are provided herein. However, these can share substantially a same geometry according to a stock size. As an example, the surgeon has the option intraoperatively switching from a non-cemented apparatus of a first stock size to a cemented apparatus of the same first stock size. This can reduce surgical complexity and time.

Additional features and benefits of the various examples provided herein will be discussed and/or will be apparent to one of ordinary skill in the art.

To further illustrate the apparatuses, systems and methods disclosed herein, the following non-limiting examples are provided, and which are referred to below as techniques. Parts or all of these examples/techniques can be combined in any manner.

The invention relates to a tibial prosthesis as set out in claim 1. Optional features are set out in the dependent claims.

### BRIEF DESCRIPTION OF THE DRAWINGS

In the drawings, which are not necessarily drawn to scale, like numerals can describe similar components in different views. Like numerals having different letter suffixes can represent different instances of similar components. The drawings illustrate generally, by way of example, but not by way of limitation, various examples discussed in the present document.
FIGS. 1A-1E are plan views of a tibial baseplate from various perspectives according to an example of the present application.
FIGS. 1F-1K are various cross-sectional views of the tibial baseplate of FIGS. 1A-1E according to an example of the present application.
FIG. 2 illustrates a system including a plurality of tibial baseplates with keels of different proximal-distal lengths according to an example of the present application.
FIG. 2A is a chart of changes in the proximal-distal lengths of the keel as compared with a standard size of the tibial baseplate according to an example of the present application.
FIGS. 3A-8B shows a system not forming part of the present invention that provides for various sizes of tibial baseplates including those configured for both non-cemented and cemented fixation to the tibia, the tibial baseplates can have a same standard size (both non-cemented and cemented versions) and can share a substantially same distal profile with respect to the keel and plurality of fins according to an example of the present application.

### DETAILED DESCRIPTION

The present application relates tibial prostheses, in particular, tibial baseplates and systems. This application focuses on distal aspects and features of the tibial baseplate as further discussed herein. As discussed previously, these distal features can improve fixation and the durability of the tibial baseplate among other benefits.

As used herein, the terms "proximal" and "distal" should be given their generally understood anatomical interpretation. The term "proximal" refers to a direction generally toward the torso of a patient, and "distal" refers to the opposite direction of proximal, i.e., away from the torso of a patient. It should be understood that the use of the terms "proximal" and "distal" should be interpreted as though the patient were standing with the knee joint in extension despite the apparatuses described herein generally being used with the knee joint in flexion. The intent is to differentiate the terms "proximal" and "distal" from the terms "anterior" and "posterior". As used herein, the terms "anterior" and "posterior" should be given their generally understood anatomical interpretation. Thus, "posterior" refers to a rear of the patient, e.g., a back of the knee. Similarly, "anterior" refers to a front of the patient, e.g., a front of the knee. Thus, "posterior" refers to the opposite direction of "anterior". Similarly, the term "lateral" refers to the opposite direction of "medial". The term "medial-lateral" means medial to lateral or lateral to medial. The term "proximal-distal" means proximal to distal or distal to proximal. The term "anterior-posterior" means anterior to posterior or posterior to anterior.

As used herein, the "periphery" of a tibial prosthesis refers to any periphery as viewed in a top plan view, e.g., in a generally transverse anatomical plane. Alternatively, the periphery of a tibial prosthesis may be any periphery as viewed in bottom plan view, e.g., in a generally transverse plane and looking at the distal surface adapted to contact a resected proximal surface of a tibial bone. In the context of a prosthesis, such as tibial baseplate described below, "home axis" refers to an axis oriented with respect to baseplate such that the baseplate home axis of baseplate is aligned with a home axis of tibia after implantation of baseplate in a proper rotational and spatial orientation. With some baseplate designs including the one illustrated herein, the home axis bisects a PCL cutout at the posterior edge of periphery of tibial plateau, and bisects anterior edge at the anterior edge of periphery of tibial plateau. It is contemplated that home axis may be oriented to other baseplate features, it being understood home axis of baseplate is positioned such that that proper alignment and orientation of baseplate upon tibia positions the home axis of baseplate coincident with home axis of tibia.

Home axis of tibial baseplate may be said to be an anteroposterior axis, as the home axis extends generally anteriorly and posteriorly when baseplate is implanted upon tibia. Tibial baseplate also defines mediolateral axis, which lies along the longest line segment contained within periphery that is also perpendicular to home axis of baseplate. As described below, the home axis and mediolateral axis cooperate to define a coordinate system useful for quantifying certain baseplate features in accordance with the present disclosure. Additionally, distal features such as the keel and the one or more fins can be aligned with respect to the anteroposterior axis, the mediolateral axis or other axes such as an axis of symmetry, a proximal-distal axis, etc.

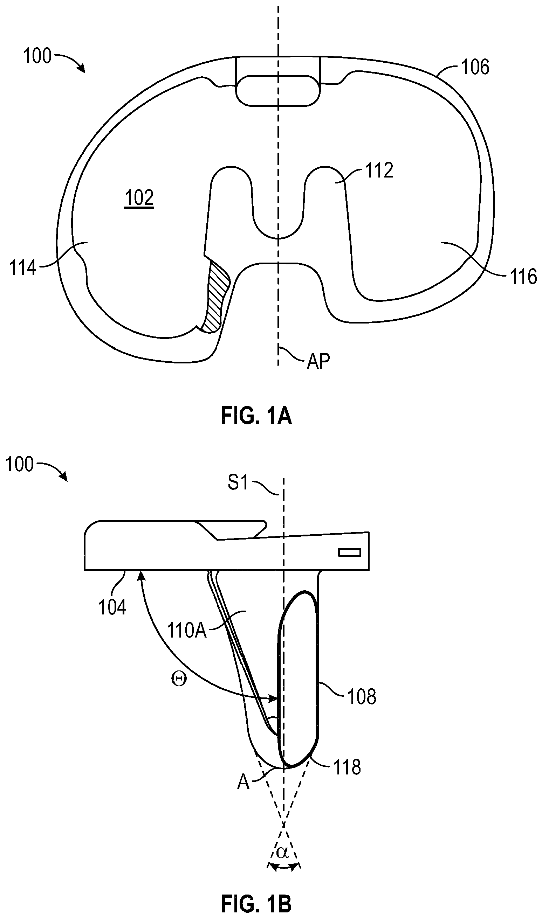

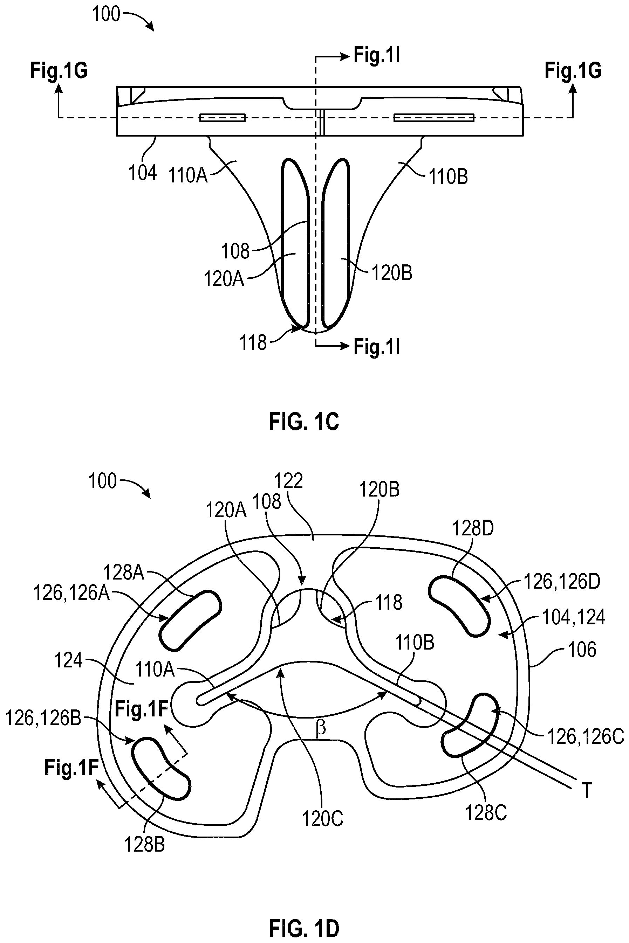

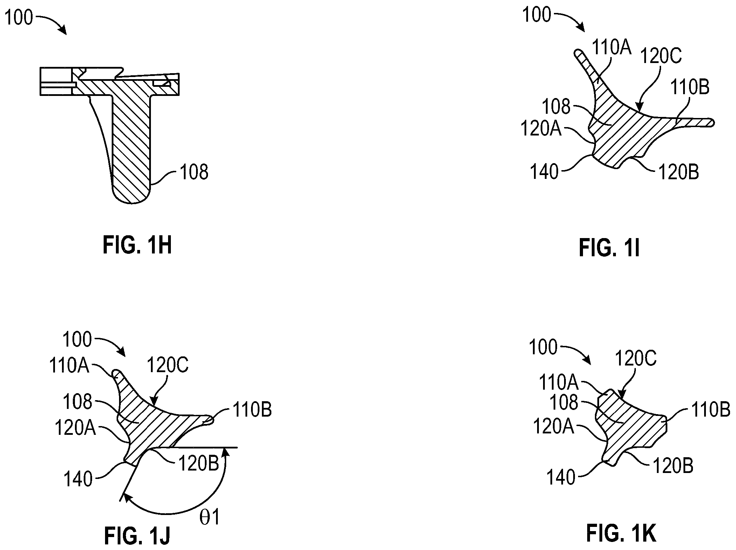

FIGS. 1A-1E show plan views of various sides of a tibial baseplate 100. The tibial baseplate 100 includes a proximal surface 102, a distal surface 104, a periphery 106. As shown in FIGS. 1B-1E the tibial baseplate 100 includes a keel 108, and a plurality of fins 110A and 110B (both shown in FIG. 1D).

FIG. 1A is a plan view of a proximal side of the tibial baseplate 100 showing the proximal surface 102, anteroposterior axis AP and periphery 106. Coupling features 112 such as bosses, rails, notches, and/or other features for coupling with a tibial bearing component (not shown) can be utilized on the proximal side. The coupling features 112 construction can be similar to that of the commercially available Persona^{®} Total Knee System from Zimmer Biomet Inc. of Warsaw, Indiana.

Tibial baseplate 100 can have a particular asymmetry, with respect to home axis and anteroposterior axis AP. This shape is designed to maximize tibial coverage for a large proportion of knee-replacement candidates as discussed in various of the Applicant's previously filed applications including United States Patent Application Publication Nos. 2013/0024001A1 and 2013/0131820A1. Maximized coverage of cortical bone facilitates superior support of tibial baseplate 100. A firm, enduring fixation of tibial baseplate 100 to tibia is facilitated by largearea contact between the cortical and cancellous bone of tibia.

The anteroposterior axis AP can divide the tibial baseplate 100 into a medial compartment 114 and a lateral compartment 116. The medial compartment 114 can be asymmetrically shaped and sized with respect to the lateral compartment 116 as defined along the periphery 106. The periphery 106 can be shaped as a wall and can have the coupling features 112 there along at one or more select locations.

The tibial baseplate 100 can be available in a plurality of standard sizes. These standard sizes are selected to provide coverage to various sizes of tibia. As an example, the tibial baseplate 100 can include nine sizes from a size 1 (small stature) to a size 9 (large stature). The various sizes can be available as a system to a surgeon to make a proper size election. The standard sizes have different geometries including along the proximal side (e.g., periphery 106, proximal surface 102, etc.) and distal side (e.g., distal surface 104, keel 108, and the plurality of fins 110A and 110B, etc.) as further described herein.

FIGS. 1B-1E show further plan views of the tibial baseplate 100 from various sides. FIG. 1B shows the keel 108 and one of the plurality of fins 110A. The fin 110A extends between the keel 108 and the distal surface 104. The keel 108 extends generally distally from the distal surface 104 and remainder of the tibial baseplate 100. The fins 110A are part of the keel 108 assembly but are described herein as distinct features from a remainder of the keel 108 that includes a stem feature. The keel 108 can be at an angle Θ with regard to the distal surface 104 (as measured from the distal surface 104 to an axis of symmetry S1 for the keel 108). According to the example of FIG. 1A, the angle Θ can substantially 90 degrees. The axis of symmetry S1 of the keel 108 can be a proximal-distal axis.

FIG. 1B shows the keel 108 can include the stem feature having a distal tip 118. This distal tip 118 can be shaped for insertion into a canal of the tibia. Thus, the distal tip 118 can be shaped as dome or hemisphere or otherwise shaped to lack sharp edges, for example. The surfaces that form the distal tip 118 can have a geometry to form an angle α of between 30 degrees and 70 degrees (substantially 50 degrees preferred). The surfaces that form the distal tip 118 can be curved and can have a radius of curvature of between 3 mm and 6 mm, for example. According to one example the radius of curvature can be substantially 4.5 mm, for example. The distal tip 118 can include an apex, for example. It should be noted that the keel 108 with the distal tip 118 is not configured for coupling with a stem extension or other feature as is common in some total knee systems. Rather, the keel 108 generally increases in proximal-distal length with increases in the standard size of the tibial baseplate 100.

FIGS. 1C and 1D show the keel 108 and the plurality of fins 110A and 110B extending from the distal surface 104. The fins 110A and 110B can have a symmetric arrangement and mirror geometry with respect to one another and with respect to the keel 108 arranged upon opposing medial and lateral sides thereof. Although two fins 110A and 110B are shown in FIGS. 1C and 1D, other examples contemplate three or more fins. The keel 108 includes a first groove comprising a first flute120A and a second groove comprising a second flute 120B. The first flute 120A and the second flute 120B can extend from a few millimeters from the distal surface 104 to the distal tip 118. Thus, the first flute 120A and the second flute 120B can extend a majority of the proximal-distal length of the keel 108 (e.g., from 50% of the length to 95%). The first flute 120A and the second flute 120B can be generally positioned on an anterior side of the keel 108 so as to be anterior facing. The first flute 120A and the second flute 120B can be symmetrically shaped with respect to one another. The geometry of the first flute 120A and the second flute 120B will be further discussed subsequently.

The keel 108 and the fins 110A and 110B additionally form a third flute 120C (FIG. 1D) or recessed area along a posterior side of the keel 108 and the fins 110A and 110B. The third flute 120C is formed by the fins 110A and 110B, which are angled relative to one another to form an angle β. In particular, the fins 110A and 110B extend not only proximal-distal (as keel 108 in some examples) but additionally extend medial-lateral and anterior-posterior. The angle β can be between substantially 105 degrees and 140 degrees, for example. According to one example, the angle β can be substantially 126 degrees. The fins 110A, 110B can have a radius of curvature relative to the keel 108 along the posterior side thereof. This radius of curvature can be between substantially 5 mm and 10 mm. According to one example, the radius of curvature can be substantially 7.6 mm. A thickness T (FIG. 1D) of the fins 110A and 110B can vary as desired.

Referring now to FIG. 1D, the distal surface 104 can include a cement mantle 122. It further includes a first pocket 124 and a second pocket 126. The mantle 122 can surround the keel 108 and the fins 110A and 110B and can extend around the periphery 106. The first pocket 124 can actually be two separate portions (medial and lateral) separated by the mantle 122. The first pocket 124 can be recessed between substantially 0.25 mm and substantially 2.0 mm from the mantle 122. The first pocket 124 can have all edges thereof curved so as to avoid sharp corners. This facilitate the flow of bone cement to fill the first pocket 124 upon installation on the tibia.

The second pocket 126 can include a plurality of pockets 126A, 126B, 126C and 126D configured to receive bone cement. The pockets 126A, 126B, 126C and 126D can be bean shaped or otherwise curved in shape. The pockets 126A, 126B, 126C and 126D can be located adjacent (within 5 mm of) the anterior-medial, posterior-medial, posterior-lateral and anterior-lateral corners of the periphery 106.

The pockets 126A, 126B, 126C and 126D can have a respective outer edge 128A, 128B, 128C and 128D closest to the periphery 106. This edge 128A, 128B, 128C and 128D can have a shape curved or otherwise corresponding to a shape of the periphery 106 adjacent the 126A, 126B, 128C and 128D. Put another way, the edge 128A can be curved to correspond to a curvature of the periphery 106 at the anterior-medial corner. Similarly, the edge 128B can be curved to correspond to a curvature of the periphery 106 at the posterior-medial corner. The edge 128C can be curved to correspond to a curvature of the periphery 106 at the posterior-lateral corner. The edge 128D can be curved to correspond to a curvature of the periphery 106 at the anterior-lateral corner.

The second pocket 126 can be between 1.5 mm and 5 mm in depth from the mantle 122. Thus, the second pocket 126 can be recessed between 0.5 mm and 3.5 mm from the bottom (proximal surface) of the first pocket 124. The size, shape, position and number of the second pocket 126 can vary from the pockets 126A, 126B, 126C and 126D. Thus, the second pockets 126A, 126B, 126C and 126D shown are exemplary. According to one example, the second pocket 126 can be elongate in a length dimension relative to a width. Thus, the second pocket 126 can have a width of between 2.0 mm and 10 mm, a length of between 4.0 mm and 25 mm, and a radius of curvature along each opposing end thereof.

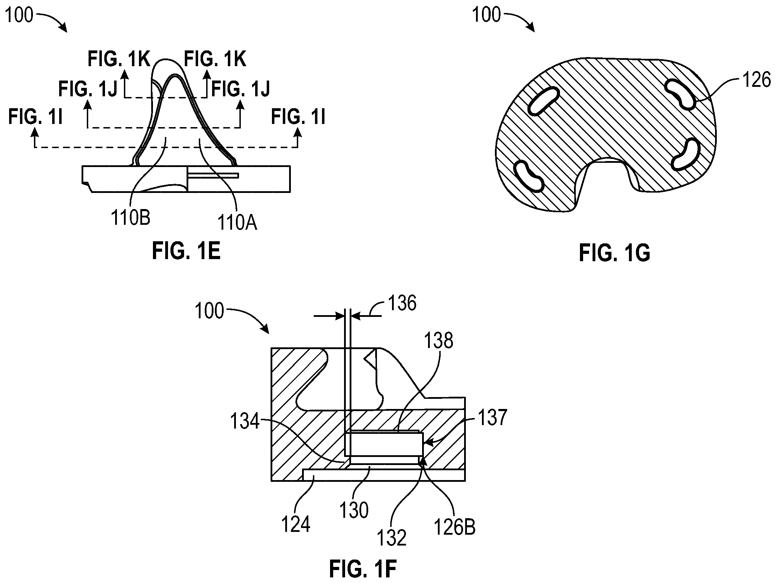

FIG. 1E shows a plan view of the tibial baseplate 100 from a posterior-medial or posterior-lateral side showing both the plurality of fins 110A and 110B.

FIG. 1F is an enlarged cross-sectional view of the second pocket 126B and other features of the tibial baseplate 100. The second pocket 126B (and indeed any of the pockets 126A, 126C and 126D) can include an opening 130, chamfer 132, a projection 134, and undercut region 136, a cavity 137 and a base surface 138.

The opening 130 can allow for communication of bone cement or other material from the first pocket 124. The chamfer 132 can define the opening 130 and can extend to the projection 134. The projection 134 can extend over the undercut region 136, which can be part of the cavity 137. The base surface 138 can comprise a proximal surface (upon implantation) defining a bottom of the cavity 137. Bone cement can flow over through the opening 130 and into the cavity 137 through a restriction created by the projection 134. Flow can be into the cavity 137 including the undercut region 136 before setting. The shape of the undercut region 136 and the projection 134 can aid the bone cement in resisting lift-off, torsion and other forces transmitted to the tibial baseplate 100. The second pocket 126 provides for additional cavity to receive bone cement to provide for additional fixation.

FIGS. 1G and 1H show further cross-sections through the baseplate 100 such as between the first pocket 124 (FIG. 1D) and the second pocket 126 (FIG. 1G) and through the keel 108 (FIG. 1H). FIG. 1H shows a true shape of the keel 108 without the fins.

FIGS. 11-1K show cross-sections of the keel 108 and the plurality of fins 110A and 110B showing features such as the first flute 120A, the second flute 120B and the third flute 120C. As shown in FIGS. 11-1K the keel and the plurality of fins 110A and 110B can taper from adjacent the distal surface to the distal tip. The first flute 120A and the second flute 120B are separated by an anterior fin 140. The anterior fin 140 can form an anterior-most portion of the keel 108 and can have a substantially uniform width for at least a majority of the proximal-distal length of the keel 108. The width of the anterior fin 140 can be on the order of 1 mm to about 3 mm, for example for the majority of the longitudinal length thereof. The width of the anterior fin 140 can increase adjacent a proximal portion of the keel 108 as shown in FIG. 1I. The first flute 120A can have an angle θ1 of between about 100 degrees and about 140 degrees. According to one example, the angle θ1 can be about 117 degrees. Similarly, the second flute 120B can have the same angle θ1 of between about 100 degrees and about 140 degrees. According to one example, the angle θ1 can be about 117 degrees. Keel 108 may not have a taper but can be substantially un-tapered along at least a majority or more of a proximal-distal length thereof. The fins 110A and 110B can be tapered as previously discussed.

According to one example, the keel 108 and the fins 110A and 110B are configured to create a press-fit with a tibial canal such as at or adjacent the first flute 120A, the second flute 120B and/or the anterior fin 140. Thus, the keel 108 and the fins 110A and 110B are configured such that a cement mantle does not extend fully around the keel 108 and/or the fins 110A and 110B with respect to the tibial canal. Rather, a press-fit retention to the bone is utilized in some locations (e.g., at or adjacent the first flute 120A, the second flute 120B and/or the anterior fin 140) and the cement mantle is used at other locations.

The fins 110A and 110B are monolithically or integrally formed with the remainder of the tibial baseplate 100 including the distal surface 104 and the keel 108. Thus, the fins 110A and 110B are features of the keel 108 as discussed above. The keel 108 and/or the fins 110A and 110B can be formed (with or without one another and with or without a remainder of the tibial baseplate 100) by additive manufacturing such by laser sintering or the like.

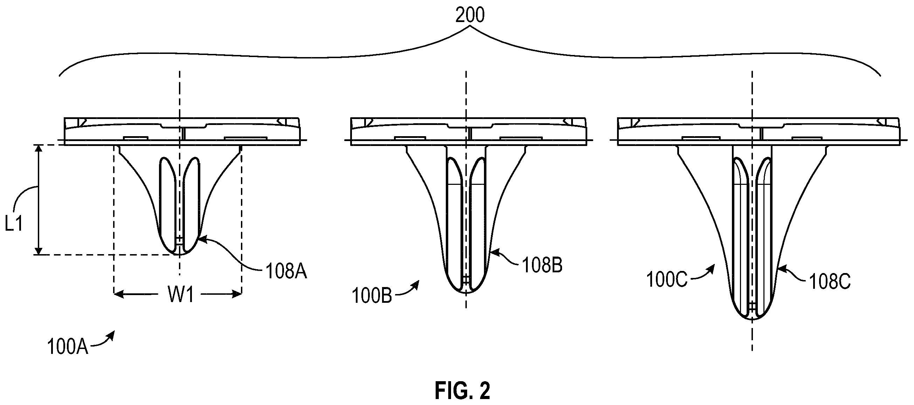

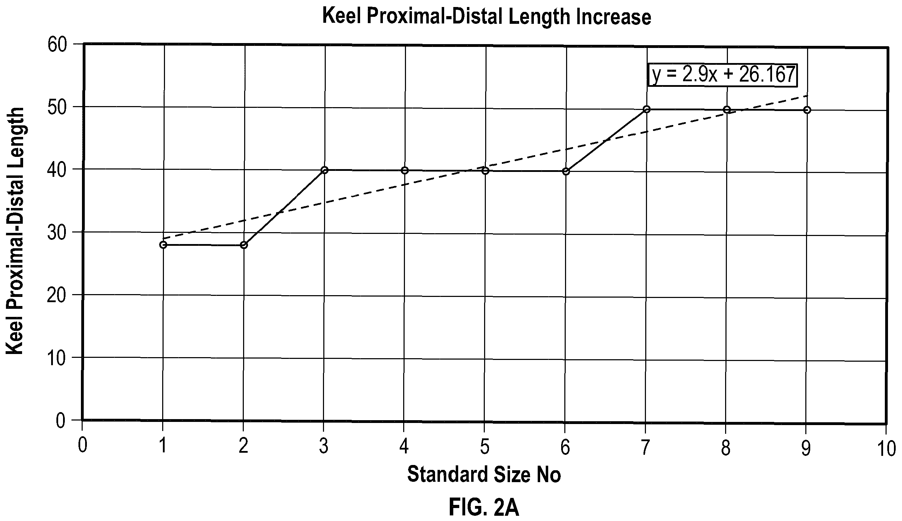

FIG. 2 shows a system 200 that includes various sizes of tibial baseplates 100A, 100B and 100C. The tibial baseplate 100A can be a standard size 1 configured for a patient with a small statured knee. The tibial baseplate 100B can be a standard size 5 configured for a patient with a middle statured knee. The tibial baseplate 100C can be a standard size 8 configured for a patient with a larger statured knee. The tibial baseplates 100A, 100B and 100C can be configured to provide a desired amount of coverage to the resected tibia without overhang or other undesired mounting arrangement. The tibial baseplates 100A, 100B and 100C can differ in regards to dimensions such as a medial-lateral extent (e.g. as measured from a first medial most edge of the periphery to a second lateral most edge), anterior-posterior extent (e.g. as measured from a third anterior most edge of the periphery to a fourth posterior most edge). However, a medial-lateral width W1 of the tibial baseplate 100A may only vary from that of the tibial baseplate 100B or tibial baseplate 100C by 20 mm or less, for example. Thus, the medial-lateral width W1 may not grow with changes in standard size in a manner commiserate with other dimensions (e.g., the medial-lateral extent of the periphery, the anterior-posterior extent of the periphery, etc.). A proximal-distal length L1 of the tibial baseplate 100A may only vary from that of the tibial baseplate 100B or tibial baseplate 100C by a substantial amount as shown in FIGS. 2 and 2A

FIG. 2 shows the tibial baseplates 100A, 100B and 100C are all nonstemmable in construction (although stemmable constructs are contemplated). Thus, a proximal-distal length of the keel 108A increases generally with an increase in standard size. Thus, the proximal distal length of the keel 108A of the tibial baseplate 100A is relatively shorter in extent than that of the keel 108B and 108C. FIG. 2A shows the increase in the proximal-distal length of the keel 108A can be step-wise increase with two or more of the different standard sizes each sharing a same proximal-distal length for the family of standard sizes. Put another way, the proximal-distal length does not increase linearly in a straight line manner with increase in a standard size but increases in a step-wise manner. Thus, at least two of the standard sizes (and sometimes up to four of the standard sizes) share the same proximal-distal length.

Exemplary lengths for the keel per standard size are provided in FIG. 2A. It should be noted the keel length does not smoothly transition in a straight line manner but has a step-functionality where several sizes (sizes 1-2, sizes-3-6 and sizes 7-9) can share substantially a same proximal-distal length. However, the general trend as shown by best fit curve is a linear increase in the proximal-distal length of the keel with change in size. It should be noted that other features discussed herein such as angles of flutes, flute radius, flute medial-lateral width, anterior fin size (medial-lateral width) and/or fin radius, posterior flute angle (e.g. angle between the fins), distal tip of keel geometry, etc. can remain substantially constant in geometry with changes in the standard size. Thus, for example a standard size 1 tibial baseplate 100A will have the same size (medial-lateral) anterior grooves as that of a standard size 8 tibial baseplate 100C (see FIG. 2).

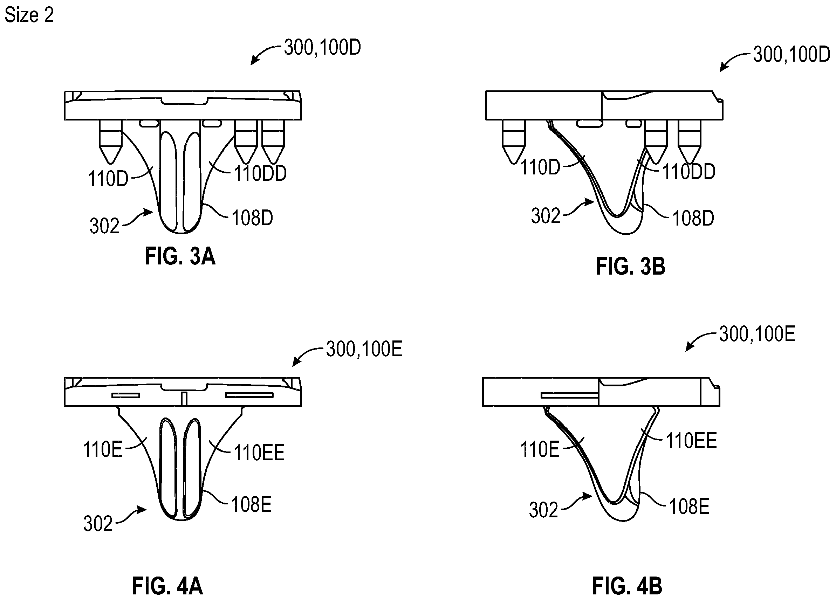

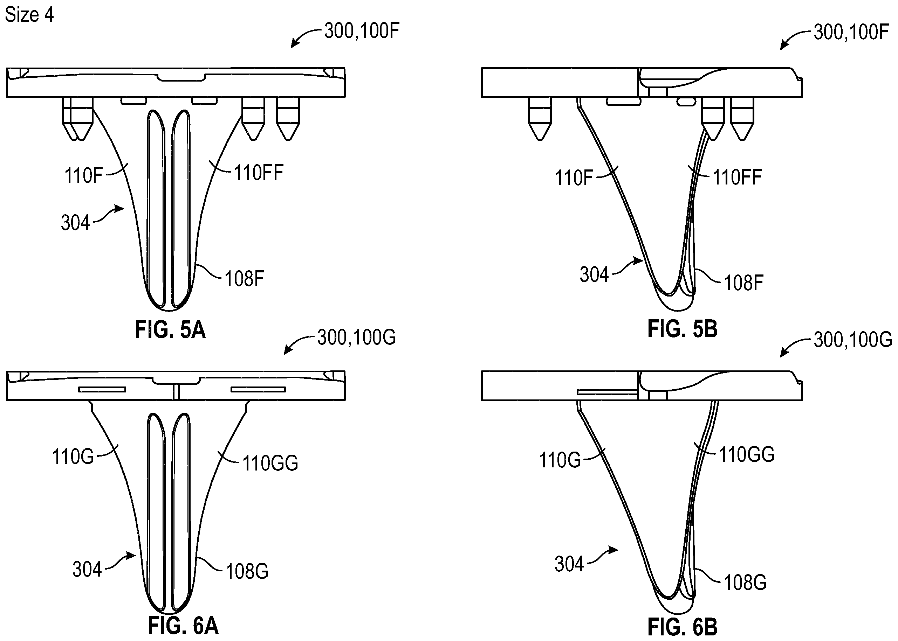

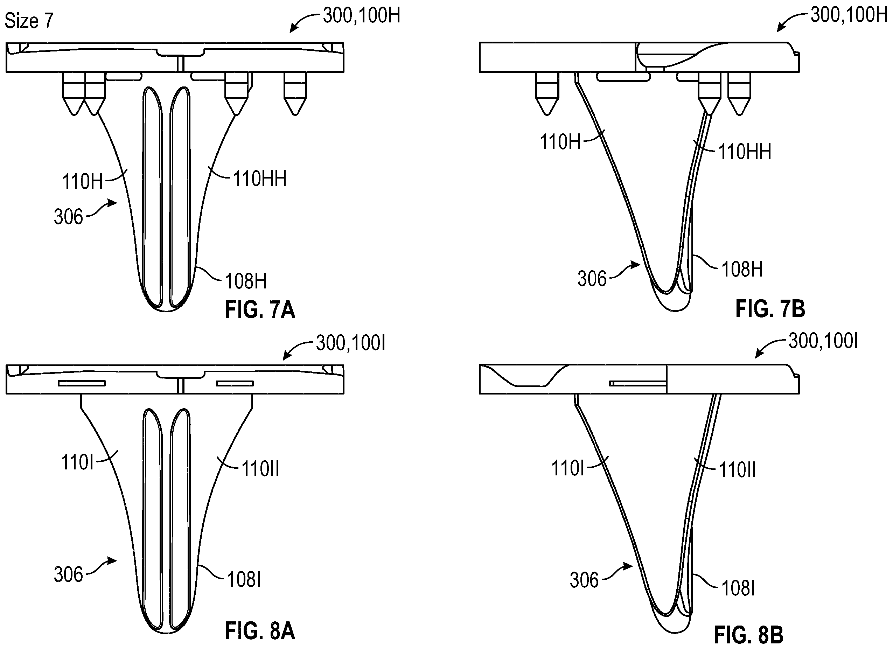

FIGS. 3A-8B show an example of a system 300 of tibial baseplates, the system not forming part of the present invention. In particular, the system 300 includes, for example, tibial baseplate 100D (FIGS. 3A and 3B), tibial baseplate 100E (FIGS. 4A and 4B), tibial baseplate 100F (FIGS. 5A and 5B), tibial baseplate 100G (FIGS. 6A and 6B), tibial baseplate 100H (FIGS. 7A and 7B) and tibial baseplate 100I (FIGS. 8A and 8B).

The tibial baseplates 100D and 100E can be of a same standard size (e.g., a standard size 2) but of a different construction in that the tibial baseplate 100D is configured for uncemented fixation (rather utilizing bone ingrowth) as compared with the tibial baseplate 100E, which is configured for cemented fixation. Similarly, the tibial baseplates 100F and 100G can be of a same standard size (e.g., a standard size 4) but of a different construction in that the tibial baseplate 100F is configured for uncemented fixation (rather utilizing bone ingrowth) as compared with the tibial baseplate 100G, which is configured for cemented fixation. The tibial baseplates 100H and 100I can be of a same standard size (e.g., a standard size 7) but of a different construction in that the tibial baseplate 100H is configured for uncemented fixation (rather utilizing bone ingrowth) as compared with the tibial baseplate 100I, which is configured for cemented fixation.

The tibial baseplates 100D and 100E can share substantially a same size (e.g., the medial-lateral extent of the periphery can be substantially the same, the anterior-posterior extent of the periphery can be substantially the same, etc.). Additionally, the geometry of the tibial baseplates 100D and 100E distal features can also be substantially the same. This includes geometry of a plurality of aspects for the keel 108D and the fins 110D, 110DD of the tibial baseplate 100D as compared with corresponding aspects for the keel 108E and the fins 110E, 110EE of the tibial baseplate 100E. Thus, as shown in FIGS. 3A-4B, the keel 108D and the fins 110D, 110DD of the tibial baseplate 100D can share substantially a same distal profile 302 (as measured along distal-most surfaces of the keel 108D and the fins 110D, 110DD moving medial-lateral along an extent of the keel 108D and the fins 110D, 110DD) as the keel 108E and the fins 110E, 110EE of the tibial baseplate 100E. Put another way, the keels 108D and 108E can share same geometric features such as but not limited to proximal-distal length, diameter, groove shape and size, flute size and shape, thickness, etc. Similarly, the fins 110D, 110DD can share a same geometry (angulation, medial-lateral width, proximal-distal length, etc.) as the fins 110E, 110EE. Such substantial match in shapes for the keel 108D and the fins 110D, 110DD with the keel 108E and the fins 110E, 110EE can allow the surgeon to choose intraoperatively between a cemented apparatus or a non-cemented apparatus. In this manner, reaming or other surgical steps that would otherwise need to be carried out to switch from a cemented apparatus to a non-cemented apparatus (or vice versa) need not be performed saving time and complexity. According to some examples, the fins 110D, 110DD and the fins 110E, 110EE may only differ in a thickness of the fins 110D, 110DD may be slightly greater than a corresponding thickness of the fins 110E, 110EE.

In the manner discussed with the tibial baseplates 100D and 100E, the tibial baseplates 100F and 100G can share substantially a same size (e.g., the medial-lateral extent of the periphery can be substantially the same, the anterior-posterior extent of the periphery can be substantially the same, etc.). Additionally, the geometry of the tibial baseplates 100F and 100G distal features can also be substantially the same. This includes geometry of a plurality of aspects for the keel 108F and the fins 110F, 110FF of the tibial baseplate 100F as compared with corresponding aspects for the keel 108G and the fins 110G, 110GG of the tibial baseplate 100G. Thus, as shown in FIGS. 5A-6B, the keel 108F and the fins 110F, 110FF of the tibial baseplate 100F can share substantially a same distal profile 304 (as measured along distal-most surfaces of the keel 108F and the fins 110F, 110FF moving medial-lateral along the extent of the keel 108F and the fins 110F, 110FF) as the keel 108G and the fins 110G, 110GG of the tibial baseplate 100G. Put another way, the keels 108F and 108G can share same geometric features such as but not limited to proximal-distal length, diameter, groove shape and size, flute size and shape, etc. Similarly, the fins 110F, 110FF can share a same geometry (angulation, medial-lateral width, proximal-distal length, thickness, etc.) as the fins 110G, 110GG. According to some examples, the fins 110F, 110FF and the fins 110G, 110GG may only differ in a thickness of the fins 110F, 110FF may be slightly greater than a corresponding thickness of the fins 110G, 110GG.

The tibial baseplates 100H and 100I can share substantially a same size (e.g., the medial-lateral extent of the periphery can be substantially the same, the anterior-posterior extent of the periphery can be substantially the same, etc.). Additionally, the geometry of the tibial baseplates 100H and 100I distal features can also be substantially the same. This includes geometry of a plurality of aspects for the keel 108H and the fins 110H, 110HH of the tibial baseplate 100H as compared with corresponding aspects for the keel 108I and the fins 110I, 110II of the tibial baseplate 100I. Thus, as shown in FIGS. 7A-8B, the keel 108H and the fins 110H, 110HH of the tibial baseplate 100H can share substantially a same distal profile 306 (as measured along distal-most surfaces of the keel 108H and the fins 110H, 110HH moving medial-lateral along the extent of the keel 108H and the fins 110H, 110HH) as the keel 108I and the fins 110I, 110II of the tibial baseplate 100I. Put another way, the keels 108H and 108I can share a same geometry of features such as but not limited to proximal-distal length, diameter, groove shape and size, flute size and shape, etc. Similarly, the fins 110H, 110HH can share a same geometry (angulation, medial-lateral width, proximal-distal length, thickness, etc.) as the fins 110I, 110II. According to some examples, the fins 110H, 110HH and the fins 110I, 110II may only differ in a thickness of the fins 110H, 110HH may be slightly greater than a corresponding thickness of the fins 110I, 110II.

According to the examples provided herein, the tibial baseplate can comprise a cruciate retaining (CR) design. Thus, the tibial baseplates can have a relieve for the posterior cruciate ligament is not resected during implantation. However, other prosthesis designs are contemplated including a posterior-stabilized (PS) design, a mid-level constraint (MLC) or constrained posterior stabilized (CPS) design, and an ultra-congruent (UC) design, for example. The PS and MLC designs utilize a spine and cam as known in the art. The posterior cruciate ligament is eliminated so no relief need be provided.

### Additional Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "generally" "substantially" "about" mean within 15 percent of the value provided (±). The terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

## Claims

1. A tibial prosthesis for a knee arthroplasty comprising:
a baseplate (100) comprising:
a distal surface (104) sized and shaped to substantially cover a proximal resected surface of a tibia;
a proximal surface (102) opposite the distal surface (104), the proximal surface (102) having a lateral compartment (116) and a medial compartment (114) opposite the lateral compartment (116);
a periphery (106) extending between the distal surface (104) and the proximal surface (102);
a first pocket (124) formed in the baseplate (100) and recessed from the distal surface (104), wherein the first pocket (124) is configured to receive a bone cement therein;
at least one second pocket (126) formed in the baseplate (100) and recessed from the first pocket (124), wherein the at least one second pocket (126) is configured to receive a portion of the bone cement;
a tibial keel (108) extending distally from the distal surface (104) to define a longitudinal tibial keel axis (S1); and
a plurality of fins (110A, 110B) spanning a junction between the tibial keel (108) and the distal surface (104),
wherein the plurality of fins (110A, 110B) are angled anterior-posterior and medial-lateral to form a third flute (120C) on a posterior of the tibial keel (108);
**characterized in that**
the tibial keel (108) includes a first flute (120A) and a second flute (120B) each extending to a distal tip (118) of the tibial keel (108), wherein the first flute (120A) and the second flute (120B) are connected by an anterior fin (140) on an anterior of the tibial keel (108),
and **in that** the tibial keel (108) is configured to form a press-fit with the tibia such that a cement mantel extends around only a portion of the tibial keel (108).

2. The tibial prosthesis of claim 1, wherein the plurality of fins (110A, 110B) form an angle (β) of substantially 126 degrees for the third flute (120C), and wherein the first flute (120A) and the second flute (120B) each have an angle (θ1) of substantially less than 126 degrees.

3. The tibial prosthesis of any one of claims 1-2, wherein the at least one second pocket (126) includes an undercut (136) so that an opening (130) to the at least one second pocket (126) is smaller than a proximal surface (138) that forms a bottom of the at least one second pocket (126).

4. The tibial prosthesis of any one of claims 1-3, wherein the at least one second pocket (126) comprises four pockets (126A, 126B, 126C, 126D) located at, respectively, a posterior-medial corner, a posterior-lateral corner, an anterior-lateral corner and an anterior-medial corner.

5. The tibial prosthesis of claim 4, wherein each of the four pockets (126A, 126B, 126C, 126D) a curvature along a least an exterior facing side (128A, 128B, 128C, 128D) that substantially matches a curvature of the periphery (106) at a corresponding location.

## Patentansprüche

1. Tibiaprothese für eine Kniearthroplastie, umfassend:
eine Grundplatte (100), umfassend:
eine distale Oberfläche (104), die dimensioniert und geformt ist, um eine proximale resezierte Oberfläche einer Tibia im Wesentlichen abzudecken;
eine proximale Oberfläche (102) gegenüber der distalen Oberfläche (104), wobei die proximale Oberfläche (102) ein laterales Kompartiment (116) und ein mediales Kompartiment (114) gegenüber dem lateralen Kompartiment (116) aufweist;
eine Peripherie (106), die sich zwischen der distalen Oberfläche (104) und der proximalen Oberfläche (102) erstreckt;
eine erste Ausnehmung (124), die in der Grundplatte (100) gebildet und von der distalen Oberfläche (104) aus ausgespart ist, wobei die erste Ausnehmung (124) dazu konfiguriert ist, einen Knochenzement darin aufzunehmen;
mindestens eine zweite Ausnehmung (126), die in der Grundplatte (100) gebildet und von der ersten Ausnehmung (124) aus ausgespart ist, wobei die mindestens eine zweite Ausnehmung (126) dazu konfiguriert ist, einen Teil des Knochenzements aufzunehmen;
einen Tibiakiel (108), der sich distal von der distalen Oberfläche (104) erstreckt, um eine longitudinale Tibiakielachse (S1) zu definieren; und
eine Vielzahl von Rippen (110A, 110B), die eine Verbindungsstelle zwischen dem Tibiakiel (108) und der distalen Oberfläche (104) überspannen,
wobei die Vielzahl von Rippen (110A, 110B) anterior-posterior und medial-lateral abgewinkelt sind, um eine dritte Nut (120C) auf einer Rückseite des Tibiakiels (108) zu bilden; **dadurch gekennzeichnet, dass**
der Tibiakiel (108) eine erste Nut (120A) und eine zweite Nut (120B) beinhaltet, die sich jeweils zu einer distalen Spitze (118) des Tibiakiels (108) erstrecken, wobei die erste Nut (120A) und die zweite Nut (120B) durch eine anteriore Rippe (140) an einer Vorderseite des Tibiakiels (108) verbunden sind,
und dadurch, dass der Tibiakiel (108) dazu konfiguriert ist, eine Presspassung mit der Tibia zu bilden, sodass sich ein Zementmantel nur um einen Abschnitt des Tibiakiels (108) erstreckt.

2. Tibiaprothese nach Anspruch 1, wobei die Vielzahl von Rippen (110A, 110B) einen Winkel (β) von im Wesentlichen 126 Grad für die dritte Nut (120C) bilden und wobei die erste Nut (120A) und die zweite Nut (120B) jeweils einen Winkel (θ1) von im Wesentlichen weniger

3. Tibiaprothese nach einem der Ansprüche 1-2, wobei die mindestens eine zweite Ausnehmung (126) eine Hinterschneidung (136) beinhaltet, sodass eine Öffnung (130) zu der mindestens einen zweiten Ausnehmung (126) kleiner als eine proximale Oberfläche (138) ist, die einen Boden der mindestens einen zweiten Ausnehmung (126) bildet.

4. Tibiaprothese nach einem der Ansprüche 1-3, wobei die mindestens eine zweite Ausnehmung (126) vier Ausnehmungen (126A, 126B, 126C, 126D) umfasst, die sich jeweils an einer posterior-medialen Ecke, einer posterior-lateralen Ecke, einer anterior-lateralen Ecke und einer anterior-medialen Ecke befinden.

5. Tibiaprothese nach Anspruch 4, wobei jede der vier Ausnehmungen (126A, 126B, 126C, 126D) eine Krümmung entlang mindestens einer nach außen weisenden Seite (128A, 128B, 128C, 128D), die im Wesentlichen mit einer Krümmung der Peripherie (106) an einer entsprechenden Stelle übereinstimmt.

## Revendications

1. Prothèse tibiale destinée à une arthroplastie du genou comprenant :
une plaque de base (100) comprenant :
une surface distale (104) dimensionnée et profilée de manière à recouvrir sensiblement une surface proximale réséquée d'un tibia ;
une surface proximale (102) opposée à la surface distale (104), la surface proximale (102) comportant un compartiment latéral (116) et un compartiment médial (114) opposé au compartiment latéral (116) ;
une périphérie (106) s'étendant entre la surface distale (104) et la surface proximale (102) ;
une première poche (124) formée dans la plaque de base (100) et en retrait de la surface distale (104), ladite première poche (124) étant conçue pour recevoir un ciment osseux en son sein ;
au moins une seconde poche (126) formée dans la plaque de base (100) et en retrait de la première poche (124), ladite au moins une seconde poche (126) étant conçue pour recevoir une partie du ciment osseux ;
une quille tibiale (108) s'étendant de façon distale à partir de la surface distale (104) de manière à définir un axe de quille tibiale longitudinal (S1) ; et
une pluralité d'ailettes (110A, 110B) couvrant une jonction entre la quille tibiale (108) et la surface distale (104),
ladite pluralité d'ailettes (110A, 110B) formant un angle antéro-postérieur et médio-latéral de manière à former une troisième cannelure (120C) sur une partie postérieure de la quille tibiale (108) ;
**caractérisée en ce que** la quille tibiale (108) comprend une première cannelure (120A) et une deuxième cannelure (120B) s'étendant chacune jusqu'à la pointe distale (118) de la quille tibiale (108), ladite première cannelure (120A) et ladite deuxième cannelure (120B) étant reliées par une ailette antérieure (140) sur une partie antérieure de la quille tibiale (108), et **en ce que** la quille tibiale (108) est conçue pour former un ajustement serré avec le tibia de sorte qu'un manteau de ciment s'étende autour d'une partie seulement de la quille tibiale (108).

2. Prothèse tibiale selon la revendication 1, ladite pluralité d'ailettes (110A, 110B) formant un angle (β) de sensiblement 126 degrés pour la troisième cannelure (120C), et ladite première cannelure (120A) et ladite deuxième cannelure (120B) présentant chacune un angle (θ1) sensiblement inférieur à 126 degrés.

3. Prothèse tibiale selon l'une quelconque des revendications 1 à 2, ladite au moins une seconde poche (126) comprenant une contre-dépouille (136) de sorte qu'une ouverture (130) de ladite au moins une seconde poche (126) soit plus petite qu'une surface proximale (138) qui forme un fond de ladite au moins une seconde poche (126).

4. Prothèse tibiale selon l'une quelconque des revendications 1 à 3, ladite au moins une seconde poche (126) comprenant quatre poches (126A, 126B, 126C, 126D) situées au niveau, respectivement, d'un coin postéro-médial, d'un coin postéro-latéral, d'un coin antéro-latéral et d'un coin antéro-médial.

5. Prothèse tibiale selon la revendication 4, chacune des quatre poches (126A, 126B, 126C, 126D) une courbure le long d'au moins un côté (128A, 128B, 128C, 128D) faisant face à l'extérieur qui correspond sensiblement à une courbure de la périphérie (106) au niveau d'un emplacement correspondant.
